# DEMANDE DE BREVET EUROPEEN

(11) **EP 1 010 410 A1**
(43) Date de publication de la demande: **21.06.2000**
(21) Numéro de dépôt: 98403170.8
(22) Date de dépôt: 16.12.1998
(51) Int. Cl.: A61F 9/007

(54) **Instrument chirurgical de découpe capsulaire**

(71) Demandeur: Optisoins SA, 1950 Sion (CH)
(72) Inventeur: Zerdab, Ivan, 73000 Chambery (FR)
(74) Mandataire: Gasquet, Denis

(57) **Abrégé**

Instrument chirurgical de découpe capsulaire destiné à permettre la découpe d'un capsulorhexis dans le cristallin de l'oeil d'un patient, caractérisé en ce qu'il comporte des moyens de découpe (MD) en forme de boucle permettant de réaliser la découpe du capsulorhexis en une seule opération.

## Description

La présente invention concerne un instrument chirurgical de découpe capsulaire destiné à être utilisé lors d'opérations chirurgicales de la cataracte pour réaliser un capsulorhexis dans le cristallin.

L'opération chirurgicale de la cataracte est devenue de plus en plus courante au fil des ans grâce aux progrès des techniques chirurgicales développées par les chirurgiens. Cette opération nécessite une découpe de la capsule antérieure du cristallin que l'on appelle couramment la capsulotomie afin d'extraire la cataracte de l'enveloppe du cristallin.

On connaît dans l'art antérieur trois types de capsulotomie destinées à permettre l'extraction extracapsulaire du cristallin, ladite extraction pouvant être effectuée de manière mécanique ou par ultrason avec une technique dite de phacoémulsification. Un premier type de capsulotomie, dite en timbre-poste, consiste à pratiquer avec un kystitome des petits traits de refend effectués circulairement ; cette technique était très utilisée dans l'extraction de type extracapsulaire, c'est-à-dire mécanique, mais peu en phacoemulsification, notamment à cause de la fragilité capsulaire engendrée. Un deuxième type de capsulotomie dite 〈〈en sourire〉〉 ou 〈〈enveloppe〉〉 utilise une extraction mécanique du noyau nécessitant une incision de l'oeil de 10 à 12 mm au lieu d'une incision de 3 à 5 mm dans la technique de phacoemulsification. Cependant, cette technique utilise deux traits de refend et fragilise ainsi la capsule du cristallin. Un troisième type de capsulotomie consiste à réaliser un capsulorhexis, c'est-à-dire une découpe circulaire continue de la capsule, sans trait de refend et donc sans fragilisation. Cette technique de découpe a permis de faire considérablement évoluer l'utilisation des techniques de phacoemulsification et par là de diminuer considérablement la durée de la récupération du patient par rapport aux techniques d'extraction dites mécaniques.

A l'heure actuelle, la découpe d'un capsulorhexis s'effectue de trois manières différentes : soit à l'aide d'une aiguille tordue formant un kystitome et qui permet au chirurgien de réaliser manuellement le trait radial puis la découpe circulaire, soit à l'aide d'une pince capsulorhexis que l'on utilise une fois le trait de refend radial effectué à l'aide d'un kystitome ou avec la pince pour pincer le lambeau capsulaire et le soulever en le dirigeant de manière à obtenir une découpe circulaire, soit par une découpe thermique ou au laser de proche en proche, c'est à dire de façon discontinue.

Ces techniques de découpe du capsulorhexis de la capsule antérieur connues dans l'art antérieur et qui ont permis le développement des techniques de phacoemulsification, présentent néanmoins des inconvénients et la réalisation d'un capsulorhexis reste un geste difficile à effectuer, dont la réussite est liée à la dextérité et à l'adresse du praticien d'une part, ainsi qu'aux conditions locales rencontrées par celui-ci, c'est-à-dire la visibilité que lui procure l'oeil du patient d'autre part. Ainsi, ces techniques présentent des inconvénients liés à la difficulté de leur mise en oeuvre, à leur non reproductibilité et aux risques de complication dus aux conditions locales. Par ailleurs, les dimensions et l'apparence de la découpe sont entièrement fonction de l'habileté du praticien à gérer les conditions opératoires avec toujours un risque de trait de refend par un capsulorhexis filant en périphérie..

Ainsi, la présente invention a pour but de résoudre les inconvénients précités à l'aide de moyens sûrs, fiables, précis et faciles à mettre en oeuvre. Elle a pour objectif de présenter un instrument de découpe destiné à permettre la découpe d'un capsulorhexis de manière reproductible et précise et de taille déterminée à l'avance afin qu'il corresponde exactement à la taille de l'implant prévu, la forme de la découpe pouvant être ronde ou ovoïde. Elle permet également de rendre la découpe plus facile à effectuer, et ce quelles que soient les conditions locales, telles que la lueur pupillaire du patient par exemple. Cet instrument a également pour objectif de préserver sa solidité à la capsule une fois la découpe effectuée et d'améliorer ainsi les résultats postopératoires après ce type d'intervention chirurgicales.

Selon sa caractéristique principale, l'instrument chirurgical de découpe capsulaire qui est destiné à permettre la découpe d'un capsulorhexis dans le cristallin de l'oeil d'un patient est caractérisé en ce qu'il comporte des moyens de découpe en forme de boucle permettant de réaliser la découpe du capsulorhexis en une seule opération.

Selon une caractéristique complémentaire de l'instrument chirurgical de découpe capsulaire selon l'invention, les moyens de découpe sont mobiles entre une position active de découpe et une position escamotée inactive.

Selon une autre caractéristique, l'instrument chirurgical de découpe capsulaire comporte un organe de commande destiné à commander le mouvement des moyens de découpe entre leur position inactive et leur position active et inversement.

Selon un mode de réalisation de l'instrument chirurgical de découpe capsulaire selon l'invention, celui-ci est caractérisé en ce que les moyens de découpe sont de type mécanique et sont formés par une lame.

Selon une première variante d'exécution de l'instrument chirurgical de découpe, la lame est flexible et l'instrument comporte des moyens de guidage et de positionnement destinés à guider la lame pour lui donner la forme de boucle voulue lorsqu'elle se trouve en position active de découpe.

Selon cette variante d'exécution, les moyens de guidage et de positionnement sont constitués par un anneau formé de deux parois concentriques entre lesquelles coulisse la lame, ladite lame étant réalisée en matériau flexible.

Selon une autre variante d'exécution de l'instrument chirurgical de découpe capsulaire selon l'invention, celui-ci est caractérisé en ce que la lame est constituée par un alliage à mémoire de forme.

Selon un autre mode de réalisation de l'instrument chirurgical de découpe capsulaire selon l'invention, les moyens de découpe sont de type thermique.

D'autres caractéristiques et avantages de l'invention se dégageront de la description qui va suivre en regard des dessins annexés qui ne sont donnés qu'à titre d'exemples non limitatifs.

Les figures 1 à 7b illustrent le mode de réalisation préféré de l'instrument chirurgical de découpe d'un capsulorhexis et une variante d'exécution de ce mode de réalisation.

Les figures 1 à 4b illustrent le mode de réalisation préféré de l'instrument de découpe.
La figure 1 illustre en perspective le mode de réalisation préféré de l'instrument de découpe.
La figure 2a montre en détail en perspective les moyens de guidage et de découpe de l'instrument de découpe en position escamotée.
La figure 2b illustre dans une vue similaire à la figure 2a les moyens de guidage et de découpe en position active de découpe.
La figure 3 illustre en coupe transversale les moyens de guidage et de découpe.
La figure 4a représente en coupe longitudinale l'instrument de découpe en position active.
La figure 4b illustre dans une vue similaire à la figure 4a l'instrument de découpe en position escamotée.
Les figures 5a à 6b illustrent une variante d'exécution de l'instrument chirurgical de découpe.

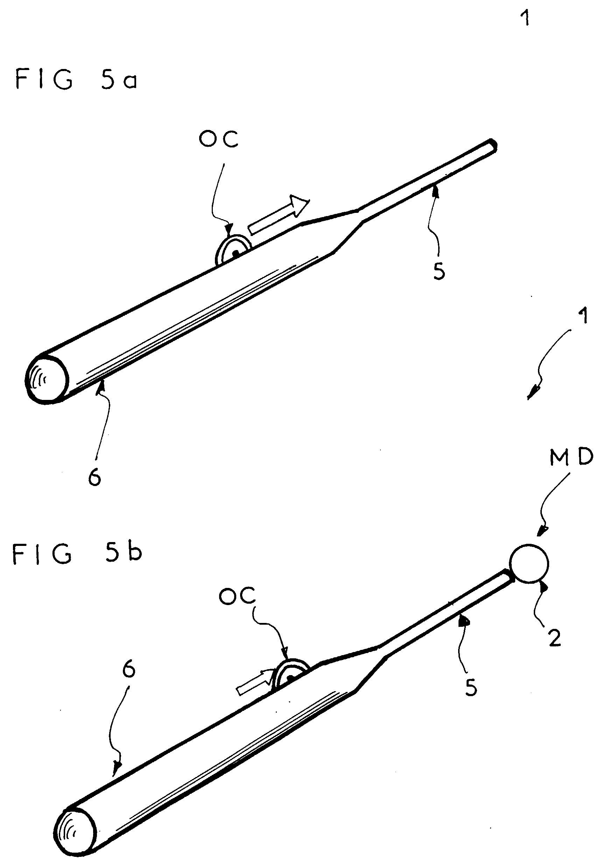

La figure 5a illustre en perspective l'instrument chirurgical de découpe en position escamotée.
La figure 5b représente dans une vue similaire à la figure 5a l'instrument de découpe en position active.

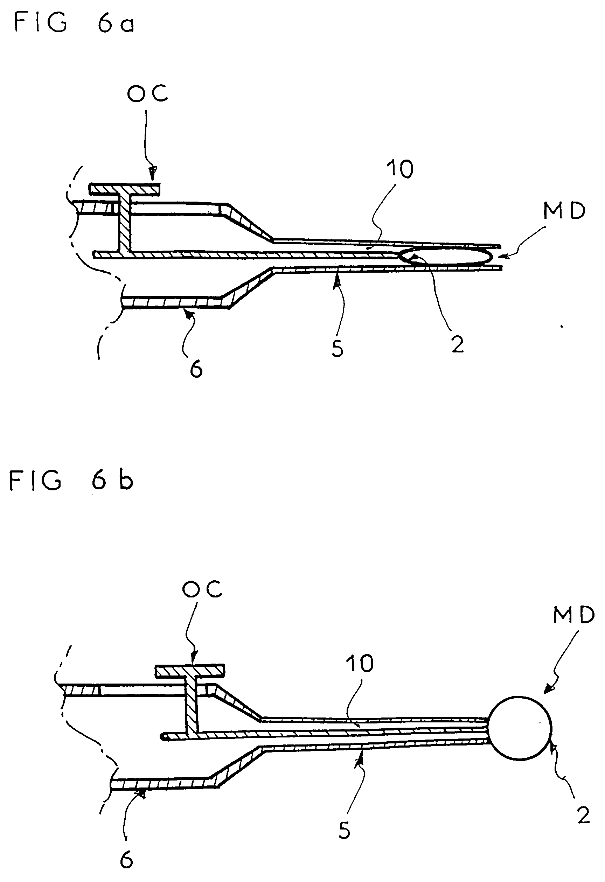

Les figures 6a et 6b illustrent respectivement en coupe longitudinale l'instrument de découpe en position escamotée et active.

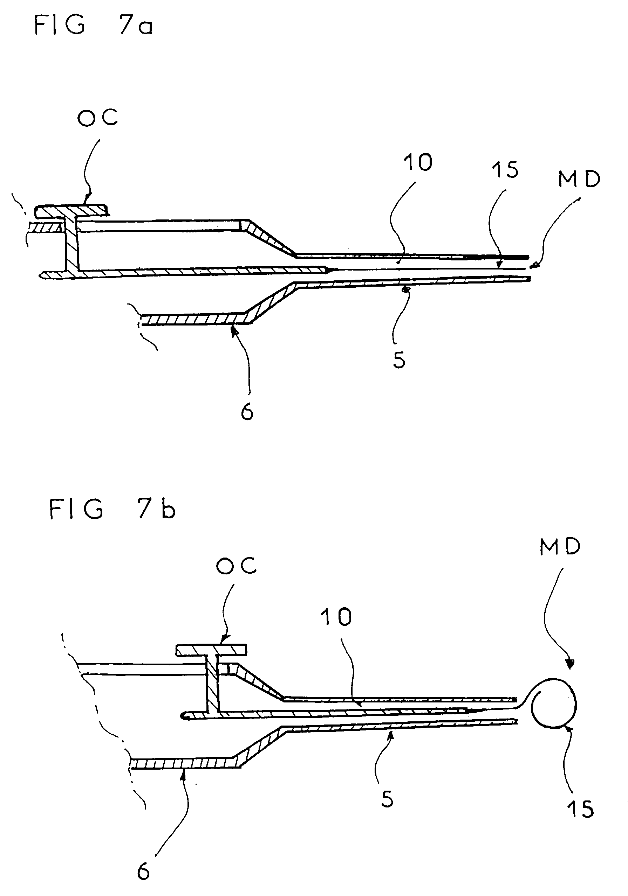

Les figures 7a et 7b illustrent respectivement en coupe longitudinale une deuxième variante d'exécution de l'instrument de découpe respectivement en position escamotée et en position active.

La présente invention concerne un instrument chirurgical de découpe capsulaire portant la référence générale (1). Il est destiné à être utilisé lors d'opérations chirurgicales, notamment de la cataracte pour réaliser un capsulorhexis dans la face antérieure de la capsule du cristallin du patient, afin de permettre notamment l'installation d'un implant dans la capsule.

Selon l'invention, l'instrument chirurgical de découpe capsulaire (1) comporte des moyens de découpe (MD) en forme de boucle qui permettent la découpe du capsulorhexis en une seule opération c'est à dire en un seul geste ou en un seul temps, le capsulorhexis est ainsi découpé d'un seul tenant. Les moyens de découpe (MD) sont avantageusement mobiles entre une position active de découpe (B) où le chirurgien peut effectuer la découpe d'un seul geste et une position inactive escamotée (A) qui permet avantageusement l'introduction de l'instrument dans l'oeil par une incision réalisée dans celui-ci, leur mouvement étant sollicité par un organe de commande (OC).

Selon l'invention, les moyens de découpe (MD) sont de type régulier et produisent ainsi une découpe parfaitement reproductible, c'est-à-dire l'obtention de capsulorhexis de forme et de dimension identiques à chaque utilisation de l'instrument (1). Selon le mode de réalisation préféré de l'instrument chirurgical de découpe les moyens de découpe (MD) sont de type circulaire de manière à obtenir une découpe en forme de cercle.

Notons que la découpe arrondie obtenue est réalisée sensiblement d'un seul tenant. Cependant, il peut rester sur le périmètre du cercle ainsi découpé un pont capsulaire, c'est-à-dire une zone ou portion de la capsule de très faible taille qui n'a pas été découpée. Toutefois, le pont capsulaire obtenu peut être éliminé par une simple traction sur le capsulorhexis réalisé à l'aide d'une pince lorsque le praticien enlève le capsulorhexis pour permettre son intervention.

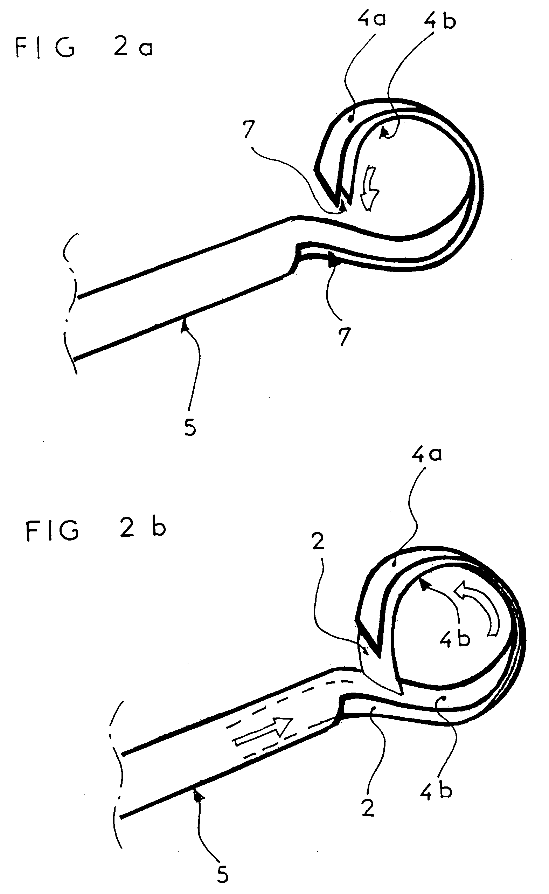

Selon le mode de réalisation préféré de l'instrument chirurgical de découpe (1), ses moyens de découpe (MD) permettent la découpe de la capsule d'un seul tenant sous la forme d'un cercle de diamètre (D) compris avantageusement entre 1 et 8 mm et pouvant être choisi égal à 5,5 mm. Lorsque le praticien actionne l'organe de commande (OC) de l'instrument (1), une fois que l'instrument est introduit à l'intérieur de l'oeil du patient, les moyens de découpe (MD) passent de leur position escamotée (A) illustrée figure 2a vers leur position active de découpe illustrée figure 2b, et le chirurgien peut ainsi disposer l'instrument de manière à positionner les moyens de découpe contre la face antérieure de la capsule du cristallin pour réaliser le capsulorhexis. Il va de soi que le diamètre (D) du cercle découpé est choisi en adéquation avec la taille de l'implant que l'on veut disposer dans la capsule et qu'il pourrait ne pas être compris dans la fourchette précitée, sans pour autant sortir du champ de protection de l'invention.

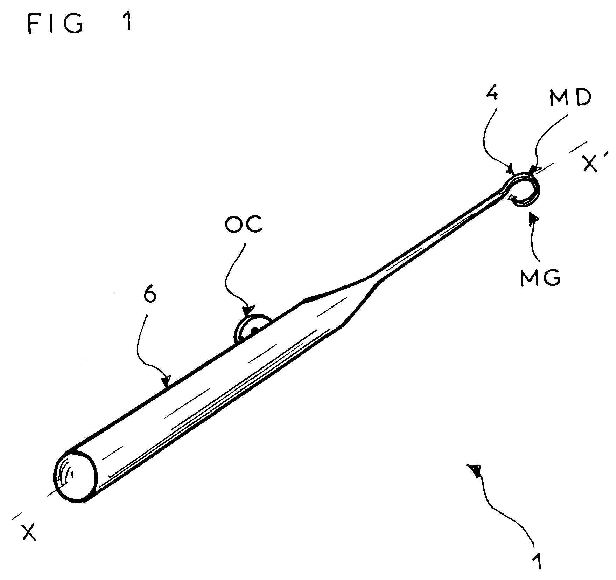

Selon un premier mode de réalisation illustré figures 1 à 4b, les moyens de découpe (MD) sont de type mécanique et sont constitués par une lame (2) possédant une forme circulaire et dont le tranchant (3) est destiné à être appuyé contre la capsule antérieure pour réaliser le capsulorhexis. Cette lame (2) est avantageusement destinée à être mise en place à l'extrémité de l'instrument (1) sous l'impulsion de l'organe de commande (OC) ou de moyens de liaisons liés à cet organe une fois que l'extrémité de l'instrument est introduit dans l'oeil.

Selon le mode de réalisation illustré et comme le montre les figures 1 et 2, l'instrument de découpe (1) possède des moyens de guidage (MG) tel qu'un anneau (4) à l'intérieur duquel vient se positionner la lame qui, guidée le long de cet anneau, vient former un cercle dont le diamètre (D) correspond à la taille voulue du capsulorhexis. Notons que l'anneau comporte avantageusement deux parois concentriques (4a, 4b) reliées entre elles le long de leur bord supérieur et qui prolongent l'extrémité d'un manchon (5) porté par le manche (6) de l'instrument. Les parois forment ainsi une fente (7) qui prolonge l'alésage interne (10) du manchon (5) qui contient la lame lorsque les moyens de découpe sont en position escamotée.

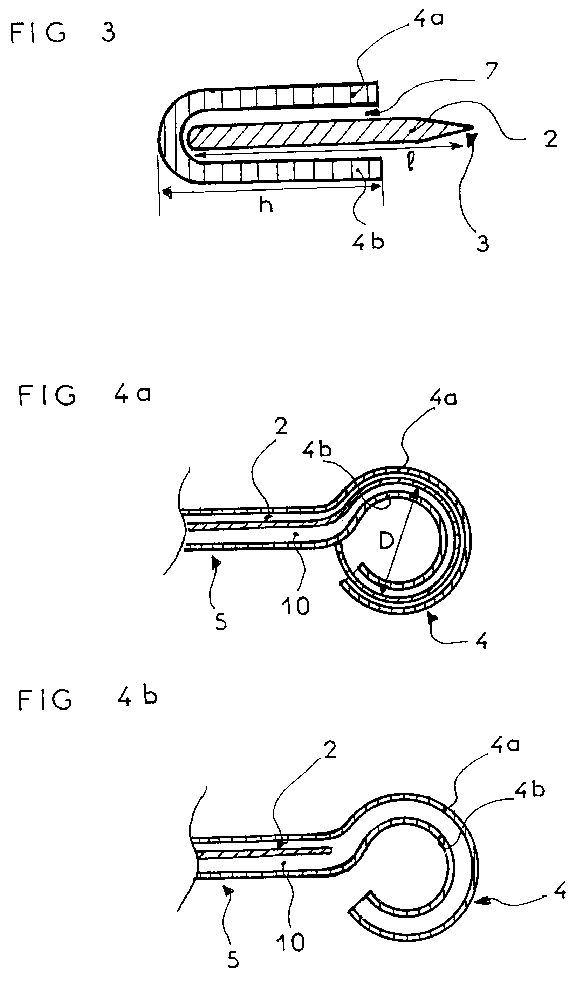

La lame (2) est avantageusement de type flexible de manière à pouvoir glisser le long de la fente (7) formée par les parois (4a, 4b) de l'anneau pour prendre la forme du cercle désiré. Notons comme le montre la figure 3, que la hauteur (h) des parois (4a, 4b) de l'anneau (4) est inférieure à la hauteur (1) de la lame (2) de manière à ce que, lorsque celle-ci vient se positionner dans l'anneau (4), son tranchant (3) s'étend au-delà du bord inférieur des parois concentriques (4a, 4b) de l'anneau.

Selon ce premier mode de réalisation, la lame (2) est mobile entre une position escamotée (A) inactive où elle est disposée à l'intérieur du manchon (5) et une position de découpe active (B) où elle est positionnée dans l'anneau (4) de manière à pouvoir être appuyée contre la face antérieure de la capsule du cristallin pour obtenir la découpe automatique du capsulorhexis. Notons que, selon ce mode de réalisation, les moyens de découpe (MD) sollicitent la capsule pour provoquer sa découpe de l'extérieur vers l'intérieur. Ainsi ils sollicitent la capsule de façon inverse à la poussée vitréenne présente en cas d'hypertonie, contrairement aux techniques de capsulorhexis de l'art antérieur qui utilisent une aiguille ou une pince à capsulorhexis et qui consistent à soulever ou à repousser le lambeau au fur et à mesure de sa découpe et qui sollicitent la capsule de l'intérieur vers l'extérieur dans le sens de la poussée vitréenne.

Selon le mode de réalisation illustré, l'organe de commande (OC) est un levier disposé coulissant selon l'axe longitudinal (XX') de l'instrument de découpe (1) et dont l'actionnement provoque le déplacement de la lame (2) entre sa position escamotée (A) et sa position active (B) par l'intermédiaire de moyens de liaison non représentés. Notons que ce levier est disposé de manière à s'étendre de manière orthogonale à l'axe longitudinal (XX') du manche (6). Toutefois, il pourrait en être autrement et l'organe de commande pourrait être constitué par un bouton poussoir disposé selon l'axe (XX') à l'extrémité du manche, par exemple, sans pour autant sortir du champ de protection de l'invention.

Selon une variante du premier mode de réalisation illustrée figure 5a à 6b, les moyens de découpe (MD) sont de type mécanique, constitués par une lame (2) ou équivalent. Toutefois, l'instrument de découpe (1) ne comporte pas de moyens de guidage, tel que l'anneau décrit précédemment. Selon cette variante, la lame (2) est réalisée dans un matériau à mémoire de forme tels que certains alliages qui lui permettent de se positionner en forme de boucle, et plus précisément de cercle, sans nécessiter de moyens de guidage lorsqu'elle passe de sa position escamotée (A) à sa position active (B).

Selon cette variante d'exécution, l'instrument (1) se présente sous une forme sensiblement identique au mode de réalisation précédemment décrit avec un manche (6) portant l'organe de commande (OC), prolongé par un manchon (5) à l'intérieur duquel se trouve la lame (2) lorsqu'elle est escamotée comme le montre la figure 6a. En position escamotée, la lame se replie, par exemple, pour prendre une forme ovoïde allongée à l'intérieur du manchon (5), tandis que sous l'impulsion directe ou indirecte de l'organe de commande (OC), elle sort à l'extrémité du manchon pour prendre la forme circulaire désirée dans sa position active de découpe (B) comme le montre la figure 6b.

L'avantage de cette variante d'exécution utilisant un matériau à mémoire de forme est de permettre une incision dans l'oeil de faible taille par rapport à celle que l'on doit effectuer pour opérer à l'aide des instruments classiques ou de l'instrument de découpe précédemment décrit qui possède l'anneau de guidage à son extrémité. Notons qu'il peut également être avantageux de réaliser les moyens de guidage à l'aide d'un matériau à mémoire de forme et de conserver ainsi des moyens de découpe classique ou autre utilisant de tels moyens de guidage.

Selon un deuxième mode de réalisation, les moyens de découpe (MD) ne sont plus de type mécanique, tel que précédemment décrit, mais sont avantageusement de type thermique. Ils sont constitués par un filament (15) disposé circulairement en position active comme le montre la figure 7b, ledit filament pouvant se mettre en position active grâce à des moyens de guidage qui peuvent être similaires à ceux précédemment décrits ou, comme le montre les figures 7a, 7b, sans nécessiter de moyens de guidage grâce à l'utilisation de matériau à mémoire de forme par exemple.

Il va de soi que d'autres types de moyens de découpe, comme ceux utilisant des lasers pourraient être envisagés pour permettre la réalisation du capsulorhexis sans pour autant sortir du champ de protection de l'invention. La configuration des moyens de découpe de l'instrument chirurgical (1) permet, quels que soient les moyens choisis, de réaliser une découpe de la face antérieure du cristallin arrondie, régulière et reproductible selon une taille prédéterminée, la découpe s'effectuant en une seule opération. Notons qu'une deuxième opération pourrait s'avérer nécessaire si un pont capsulaire persistait suite à la découpe afin de permettre son ablation éventuelle.

Selon l'invention, l'instrument chirurgical de découpe (1) peut être réalisé en deux parties dissociables : une première partie formée du manche (6) muni de son organe de commande, sur laquelle vient se monter de manière amovible à l'aide de moyens de connexion complémentaires une deuxième partie comportant les moyens de découpe proprement dits. Ainsi, sur un même manche (6), il peut être possible d'adapter plusieurs parties secondaires ou têtes de découpe différentes permettant ainsi au chirurgien d'adapter à volonté les moyens de découpe adéquats sur la partie principale de son instrument de découpe en fonction des conditions locales et de l'opération de découpe à réaliser.

Bien entendu, l'invention n'est pas limitée aux modes de réalisation décrits et représentés à titre d'exemples, mais elle comprend aussi tous les équivalents techniques ainsi que leurs combinaisons.

## Revendications

1. Instrument chirurgical de découpe capsulaire (1) destiné à permettre la découpe d'un capsulorhexis dans le cristallin de l'oeil d'un patient, caractérisé en ce qu'il comporte des moyens de découpe (MD) en forme de boucle permettant de réaliser la découpe du capsulorhexis en une seule opération.

2. Instrument chirurgical de découpe capsulaire (1) selon la revendication 1, caractérisé en ce que les moyens de découpe (MD) sont mobiles entre une position active de découpe (B) et une position escamotée inactive (A).

3. Instrument chirurgical de découpe capsulaire (1) selon la revendication 2, caractérisé en ce qu'il comporte un organe de commande (OC) destiné à commander le mouvement des moyens de découpe (MD) entre leur position inactive (A) et leur position active (B) et inversement.

4. Instrument chirurgical de découpe capsulaire (1) selon l'une quelconque des revendications précédentes, caractérisé en ce que les moyens de découpe (MD) sont de type mécanique et sont formés par une lame (2).

5. Instrument chirurgical de découpe capsulaire (1) selon les revendications 2 et 4, caractérisé en ce que la lame (2) est flexible et en ce que l'instrument comporte des moyens de guidage et de positionnement (MG) destinés à guider la lame (2) pour lui donner la forme de boucle voulue lorsqu'elle se trouve en position active de découpe.

6. Instrument chirurgical de découpe capsulaire (1) selon la revendication 5, caractérisé en ce que les moyens de guidage et de positionnement sont constitués par un anneau (4) formé de deux parois concentriques (4a, 4b) entre lesquelles coulisse la lame (2).

7. Instrument chirurgical de découpe capsulaire (1) selon la revendication 4, caractérisé en ce que la lame (2) est constituée par un alliage à mémoire de forme.

8. Instrument chirurgical de découpe capsulaire (1) selon l'une quelconque des revendications précédentes, caractérisé en ce que les moyens de découpe (MD) sont de type thermique.
